# EUROPEAN PATENT SPECIFICATION

(11) **EP 2 142 185 B1**
(45) Date of publication and mention of the grant of the patent: **15.08.2012**
(21) Application number: 08732012.3
(22) Date of filing: 12.03.2008
(51) Int. Cl.: A61K 31/15, A61K 31/198, A61K 31/275, A61K 31/407, A61P 9/02

(54) **DROXIDOPA AND PHARMACEUTICAL COMPOSITION THEREOF FOR THE TREATMENT OF NEURALLY MEDIATED HYPOTENSION**
DROXIDOPA UND PHARMAZEUTISCHE ZUSAMMENSETZUNG DAVON ZUR BEHANDLUNG VON NEURAL VERMITTELTER HYPERTONIE
DROXIDOPA ET COMPOSITION PHARMACEUTIQUE DE CELLE-CI POUR LE TRAITEMENT DE L'HYPOTENSION VÉHICULÉE PAR VOIE NEURALE

(30) Priority: 12.03.2007 US 894354 P
(43) Date of publication of application: 13.01.2010
(73) Proprietor: Chelsea Therapeutics, Inc., Charlotte, NC 28277 (US)
(72) Inventor: ROBERTS, Michael, J., Charlotte, NC 28277 (US); PEDDER, Simon, Fort Mill, SC 29715 (US)
(74) Representative: Noel, Chantal Odile
(86) International application number: PCT/US2008/056675
(87) International publication number: WO 2008/112773

(56) References cited:
- WO-A-2008/003028
- MATHIAS C J ET AL: "L-threo-dihydroxyphenylserine (L-threo-DOPS; droxidopa) in the management of neurogenic orthostatic hypotension: a multi-national, multi-center, dose-ranging study in multiple system atrophy and pure autonomic failure." CLINICAL AUTONOMIC RESEARCH : OFFICIAL JOURNAL OF THE CLINICAL AUTONOMIC RESEARCH SOCIETY AUG 2001, vol. 11, no. 4, August 2001 (2001-08), pages 235-242, XP002500633 ISSN: 0959-9851
- GOLDSTEIN DAVID S: "L-dihydroxyphenylserine (L-DOPS): A norepinephrine prodrug" CARDIOVASCULAR DRUG REVIEWS, NEVA PRESS, BRANFORD, CT, US, vol. 24, no. 3-4, 1 October 2006 (2006-10-01), pages 189-203, XP009107442 ISSN: 0897-5957
- KATO T ET AL: "STUDIES ON THE ACTIVITY OF L-THREO-3 4 DIHYDROXYPHENYLSERINE L DOPS AS A CATECHOLAMINE PRECURSOR IN THE BRAIN COMPARISON WITH THAT OF L DOPA" BIOCHEMICAL PHARMACOLOGY, PERGAMON, OXFORD, GB, vol. 36, no. 18, 1 January 1987 (1987-01-01), pages 3051-3058, XP002459988 ISSN: 0006-2952

## Description

### FIELD OF THE INVENTION

The present specification is directed to droxidopa, alone or in combination with one or more additional components, for use in the treatment of neurally mediated hypotension.

### BACKGROUND

Droxidopa is a known synthetic amino acid precursor of norepinephrine that is converted directly to norepinephrine via the action of dopa decarboxylase (DDC). Droxidopa is generally used to treat orthostatic hypotension (OH), as is reported by Mathias et al. (2001) C/in. Auton. Res. 11:235-242 and Goldstein (2006) Cardiovascular Drug Review 24:189-203, and can be categorized as an antiparkinsonian agent; however, multiple pharmacological activities have been observed with droxidopa, including the following: (1) it is directly converted to 1-norepinephrine by the action of the aromatic L-amino acid decarboxylase which is widely distributed in a living body, and thus has an effect of replenishing norepinephrine; (2) it has limited permeability through the blood-brain barrier into the brain; (3) it specifically recovers norepinephrine activated nerve functions which have decreased in the central and peripheral nervous system; and (4) it shows various actions, as norepinephrine, via the adrenaline receptors in various tissues. Kawabata et al. (1994) Br. J. Pharmacol. 111:503-508 further report that droxidopa exhibits antinociceptive activity via central α-adrenoreceptors in the mouse.

Neurally mediated hypotension (NMH) can be linked to fibromyalgia and chronic fatigue syndrome. In many individuals, however, NMH is a "stand-alone" condition (*i.e.*, presents without comorbidities). NMH is also known by the terms "the fainting reflex", neurocardiogenic syncope, vasodepressor syncope, the vaso-vagal reflex, and autonomic dysfunction, and the condition arises from an abnormal reflex interaction between the heart and the brain, both of which are usually structurally normal. This miscommunication typically results in a failure to maintain appropriate increases in heart rate and leads to dizziness and fainting after standing for prolonged periods. While it is not uncommon for prolonged standing to cause fluctuations in blood pressure, the body typically compensates for such fluctuations by increasing heart rate appropriately. For individuals with NMH, however, instead of increasing, the heart rate may remain constant, or actually drop, which prevents the necessary amount of blood from being circulated within the body, and particularly to the brain.

Individuals susceptible to NMH can experience the condition in a variety of situations. For example, NMH can occur in the following settings: after prolonged periods of quiet upright posture (such as standing in line, standing in a shower, or even sitting up for long periods); after being in a warm environment (such as in hot summer weather, a hot crowded room, or a hot shower or bath); immediately after exercise; and after emotionally stressful events (such as seeing blood or gory scenes, being scared, or being anxious). Some individuals also experience symptoms soon after eating, when blood flow has shifted to the intestinal circulation during the process of digestion. All humans are susceptible to activation of the vaso-vagal reflex that results in a lowered blood pressure (NMH); however, each person's susceptibility is affected by his or her genetic make-up, dietary factors, psychological make-up, and acute triggers such as infection and allergy. Thus, all humans do not suffer from NMH. Rather, the clinical problem of NMH occurs when there is sufficiently early triggering of this reflex to cause symptoms.

Neurally mediated hypotension is typically treated with a combination of increased salt and water intake in conjunction with drugs that regulate blood pressure. Some drugs work by allowing the kidneys to retain sodium and others block the body's response to adrenaline, which can kick-start the blood pressure abnormality. Known treatments for NMH generally require persistence, commitment, and the willingness to try several possible drugs and combinations over an extended period of time. Treatment often requires careful physician monitoring because there is a risk of serious side effects with some of the known treatments, such as elevated blood pressure, elevated sodium levels, lowered potassium levels, or depression. Examples of drugs that have been used to treat patients with NMH include fludrocortisone, beta-blockers, disopyramide, fluoxetine, sertraline, ephedrine, pseudoephedrine, theophylline, methylphenidate, and midodrine. All of these drugs can cause undesirable side effects and may not be effective to sufficiently treat NMH. Thus, there remains a need in the art for effective drug treatment of NMH.

### SUMMARY OF THE INVENTION

The present invention provides pharmaceutical compositions comprising droxidopa alone or in combination with one or more further pharmaceutically active compounds for use in the treatment of neurally mediated hypotension in accordance wiith claim 1-16.

In one embodiment, the invention comprises administering to a subject suffering from neurally mediated hypotension a pharmaceutical composition comprising a therapeutically effective amount of droxidopa.

In certain embodiments, treatment can be indicated in a subject that is suffering from neurally mediated hypotension and exhibiting a symptom known to be indicative of neurally mediated hypotension, such as dizziness, fainting, lightheadedness, fatigue, muscle aches, headaches, confusion, or nausea. In such embodiments, the method of treatment can comprise reducing or eliminating the symptom.

In further embodiments, treatment can be indicated in a subject that is suffering from neurally mediated hypotension and is known to have previously exhibited a symptom known to be indicative of neurally mediated hypotension, such as dizziness, fainting, lightheadedness, fatigue, muscle aches, headaches, confusion, or nausea. In such embodiments, the method of treatment can comprise preventing re-occurrence of the symptom.

In certain embodiments, the one or more further pharmaceutically active compounds comprise compounds useful for treatment or prevention of symptoms associated with neurally mediated hypotension. For example, such further pharmaceutically active compounds can comprise antidepressants (such as selective serotonin reuptake inhibitors, tricyclics, serotonin norepinephrine, reuptake inhibitors, norepinephrine reuptake inhibitors, and norepinephrine and dopamine reuptake inhibitors), corticosteroids, beta-blockers, serotonin receptor antagonists, serotonin receptor agonists, antiarrhythmic agents, ephedrine, pseudoephedrine, theophylline, methylplenidate, or midodrine, In specific embodiments, the invention is directed to a composition comprising droxidopa in combination with one or more antidepressants, such as fluoxotine, paroxetine, citalopram, escitalopram, fluvoxamine, sertraline, amitriptyline, nortriptyline, desipramine, trazodone, venlafaxine, duloxetine, milnacipran, ncfopam, bupropion, and combinations thereof.

The invention also comprises administering droxidopa in combination with one or more additional active agents having a complimentary activity to the activity of droxidopa. In one particular embodiment, the invention comprises administering a pharmaceutical composition comprising droxidopa and one or more DOPA decarboxylase inhibiting compounds. Preferably, the DOPA decarboxylase inhibiting compounds are selected from the group consisting of benserazide and carbidopa.

In a further embodiment, the invention comprises administering a pharmaceutical composition comprising droxidopa and one or more catechol-O-methyltransferase inhibiting compounds. Preferentially, the catechol-O-methyltransferase inhibiting compounds are selected from a specified group of compounds, such as entacapone, tolcapone, and nitecapone.

In another embodiment, the invention comprises administering a pharmaceutical composition comprising droxidopa and one or more cholinesterase inhibiting compounds. Preferentially, the cholinesterase inhibiting compounds are selected from a specified group of compounds, such as pyridostigmine, donepezil, rivastigmine, galantamine, tacrine, neostigmine, metrifonate, physostigmine, ambenonium, demarcarium, thiaphysovenine, phenscrine, edrophonium, cymscrine, and combinations thereof.

In yet another embodiment, the invention comprises administering a pharmaceutical composition comprising droxidopa and one or more monoamine oxidase inhibiting compounds. Preferentially, the monoamine oxidase inhibiting compounds are selected from a specified group of compounds, such as selegiline, moclobemide, and lazabemide.

When the droxidopa is combined with one or more additional active agents, the co-administration can be via a variety of methods. For example, the droxidopa and the additional active agent can be in the same pharmaceutical composition. In other embodiments, the droxidopa and the additional active agent can be administered in separate compositions. In such embodiments, the separated compositions can be administered at the same time or within close proximity to one another. Alternatively, the separate compositions can be administered as different times, which may be desirable to optimize the effects of the co-administered active agents.

### BRIEF DESCRIPTION OF THE DRAWING

Having thus described the invention in general terms, reference will now be made to the accompanying drawing wherein:

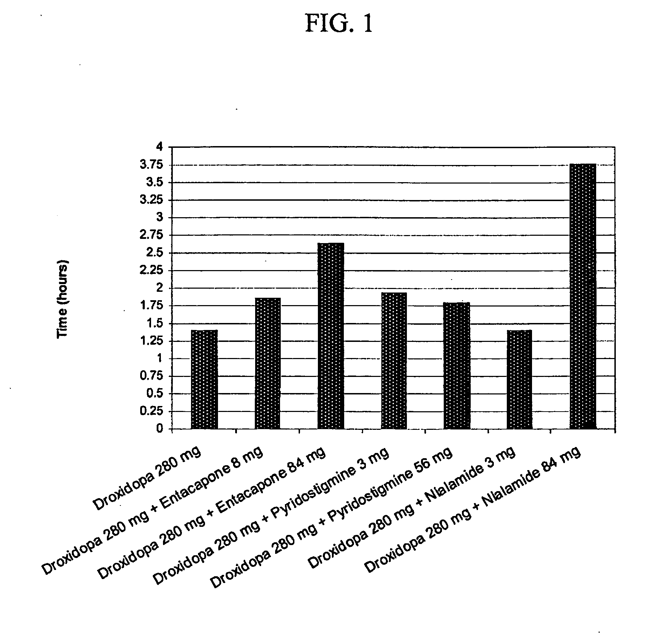

FIG. 1 is a graphical representation of the half-life of droxidopa in a mammal when administered alone or in combination with various further active agents according to certain embodiments of the invention.

### DETAILED DESCRIPTION

The invention now will be described more fully hereinafter through reference to various embodiments. These embodiments are provided so that this disclosure will be thorough and complete, and will fully convey the scope of the invention to those skilled in the art. Indeed, the invention may be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural referents unless the context clearly dictates otherwise.

The present invention provides pharmaceutical compositions for use in the treatment of neurally mediated hypotension (NMH) comprising the use of droxidopa as a single active agent. In other embodiments, treatment can comprise the use of droxidopa in combination with one or more further active agents. Such combinations are disclosed in U.S. Patent Application Publication 2008/0015181 The specific pharmaceutical composition (or compositions) used in the invention, and the methods of treatment provided by the invention, are further described below.

### 1. Active Agents

The pharmaceutical compositions of the invention generally comprise droxidopa as an active agent. In certain embodiments, the pharmaceutical compositions can comprise one or more further active agents.

### A. Droxidopa

The compositions for use in the methods of the invention generally comprisc, as an active ingredient, threo-3-(3,4-dihydroxyphenyl) serine, which is commonly known as droxidopa and has the structure provided below in Formula (I). 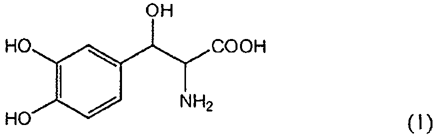 Droxidopa is also known as threo-β,3-dihydroxy-L-tyrosine, (-)-(2S,3R)-2-amino-3-hydroxy-3-(3,4-dihydroxyphenyl)propionic acid, and threo-dopaserine, as well as the common terms DOPS, threo-DOPS, and L-DOPS. The compound can is optically active and can be provided in various forms, including L-threo-DOPS, D-thrco-DOPS, L-crythro-DOPS, and D-erythro-DOPS. The compounds can also exist in the racemic form. The L-threo isomer is generally preferred according to the present invention; however, the invention also encompasses compositions and methods of use incorporating the other forms of droxidopa. Accordingly, as used throughout the present disclosure, the term "droxidopa" is intended to encompass any isolated or purified isomer (e.g., the L-threo isomer), as well as the racemic forms of droxidopa.

Droxidopa useful according to the invention can be prepared by conventional methods, including methods particularly useful for isolating the L- isomer of droxidopa. See, for example, U.S. Patent No. 3,920,728; U.S. Patent No. 4,319,040; U.S. Patent No. 4,480,109; U.S. Patent No. 4,562,263; U.S. Patent No. 4,699,879; U.S. Patent No. 5,739,387; and U.S. Patent No. 5,864,041 .

The present invention also encompasses compositions comprising one or more pharmaceutically acceptable esters, amides, salts, solvates, or prodrugs of droxidopa. In one embodiment, the invention involves use of droxidopa esters that allow for slowed or delayed decarboxylation of droxidopa resulting from hydrolytic or enzymatic degradation of the ester linkage. As would be recognized by one of skill in the art, an ester of droxidopa can be formed by replacing the hydrogen on the carboxylic ester group with any suitable ester-forming group. For example, U.S. Patent No. 5,288,898 discloses various esters of N-methylphenylserine, including methyl esters, ethyl esters, n-propyl esters, isopropyl esters, n-butyl esters, isobutyl esters, tert-butyl esters, n-pentyl esters, isopentyl esters, n-hexyl esters, and the like, and the present specification encompasses such esters, as well as other esters. Further examples of ester-forming groups that could be used according to the invention are disclosed in U.S. Patent No. 5,864,041.

### B. Additional Active Agents

As noted above, in certain embodiments, the compositions for use according to the invention can comprise one or more active agents in addition to droxidopa. Various preferred active agents that can be combined with droxidopa for treatment of neurally mediated hypotension are described below. Of course, such disclosure should not be viewed as limiting the scope of further active agents that may be combined with droxidopa. Rather, further active compounds, particularly compounds identified as useful for treating neurally mediated hypotension, or for treating or preventing symptoms associated with neurally mediated hypotension, may be used in addition to the compounds specifically disclosed herein.

In one particular embodiment, an active agent used in combination with droxidopa comprises one or more DOPA decarboxylase (DDC) inhibitors. DDC catalyzes the decarboxylation of levodopa (L-DOPA or 3,4-dihydroxy-L-phenylalanine) and 5-hydroxytryptophan (5-HTP) to yield dopamine and serotonin, respectively. Similarly, DDC catalyzes the conversion of droxidopa to pentanedione, also called nitecapone. In addition to the above example, U.S. Patent No. 6,512,136 describes various substituted 2-phenyl-1-(3,4-dihydroxy-5-nitrophenyl)-1-ethanone compounds that may also be useful as COMT inhibitors according to the present invention. Likewise, U.S. Patent No. 4,963,590; GB 2 200 109; U.S. Patent No. 6,150,412; and EP 237 929, each describes groups of COMT inhibiting compounds that could be useful according to the present invention

According to another embodiment of the invention, an active agent used in combination with droxidopa comprises one or more compounds that at least partially inhibit the function of cholinesterase. Such cholinesterase inhibiting compounds may also be referred to as anticholinesterase compounds. Cholinesterase inhibiting compounds can be reversible or non-reversible. The present invention preferably encompasses any compounds that may be considered reversible cholinesterase inhibitors (either competitive or non-competitive inhibitors). Non-reversible cholinesterase inhibitors generally find use as pesticides (such as diazinon and Sevin) and chemical weapons (such as tabin and sarin) and are not preferred according to the present invention.

Cholinesterase inhibitors are understood to include compounds that increase levels of acetylcholine (or a cholinergie agonist), generally by reducing or preventing the activity of chemicals involved in the breakdown of acetylcholine, such as acetylcholinesterase. Cholinesterase inhibitors may also include compounds having other mechanisms of action, such as stimulating release of acetylcholine, enhancing response of acetylcholine receptors, or potentiating gonadotrophin releasing hormone (GNRH)-induced growth hormone release. Moreover, cholinesterase inhibitors may act by enhancing ganglionic transmission.

Any compound generally recognized as being a cholinesterase inhibitor (or an anticholinesterase compound) may be useful according to the present invention. Non-limiting examples of cholinesterase inhibitors useful in combination with droxidopa for preparing compositions according to the invention include the following: 3-dimethylcarbamoyloxy-1-methylpyridinium, also called pyridostigmine (MESTINON® or Regonol); (±)-2,3-dihydro-5,6-dimethoxy-2-[[1-(phenylmethyl)-4-piperidinyl]methyl]-1*H*-inden-1-one, also called donepezil (ARICEPT^{®}); (S)-N-ethyl-3-((1-dimethyl-amino)ethyl)-N-methylphenyl-carbamate, also called rivastigmine (Exclon); (4aS,6R,8aS)-4a,5,9,10,11,12-hexahydro-3-methoxy-11-methyl-6H-benzofuro[3a, 3, 2ef][2]benzazepin-6-ol, also called galantamine (REMINYL^{®}) or RAZADYNE^{®}); 9-amino-1,2,3,4-tetrahydroacridine, also called tacrine (COGNEX^{®}); (*m*-hydroxyphenyl) trimethylammonium methylsulfate dimethylcarbamate, also called neostigmine; 1-hydroxy-2,2,2-triehloroethylphosphonic acid dimethyl ester, also called metrifonate or trichlorofon; 1,2,3,3A,8,8A-hexahydro-1,3a,8-trimethylpyrrolo-[2,3-b]-indole-5-ol methylcarbamate ester, also called physostigmine; [Oxalylbis(iminoethylene)]-bis-[(o-chlorobenzyl) diethylammonium] dichloride, also called ambenonium (MYTELASE^{®}); ethyl (*m*-hydroxyphenyl) dimethylammonium, also called edrophonium (ENLON^{®}); demarcarium; thiaphysovenine; phenserine; and cymscrine.

More generally, compounds useful as cholinesterase inhibitors according to the invention can comprise carbamate compounds, particularly phenylcarbamates, oganophosphate compounds, piperidines, and phenanthrine derivatives. The invention further comprises cholinesterase inhibitors that are carbamoyl esters, as disclosed in U.S. Published Patent Application No. 2005/0096387

The above groups of compounds, and specific compounds, are provided to exemplify the types of cholinesterase inhibitors that are useful according to the invention and should not be viewed as limiting the scope of the invention. In fact, the invention can incorporate various further cholinesterase inhibitors, including compounds described in the following documents Brzostowska, Malgorzata, et al. "Phenylcarbamates of (-)-Escroline, (-)-N1-Noreseroline and (-)-Physovenol: Selective Inhibitors of Acetyl and, or Butyrylcholinesterase." Medical Chemistry Research. (1992) Vol. 2, 238-246; Flippen-Anderson, Judith L., et al. "Thiaphysovenol Phenylcarbamates: X-ray Structures of Biologically Active and Inactive Anticholinesterase Agents." Heterocycles. (1993) Vol. 36, No. 1; Greig, Nigel H., et al. "Phenscrine and Ring C Hetcro-Analogues: Drug Candidates for the Treatment of Alzheimer's Disease." Medicinal Research Reviews. (1995) Vol. 15, No. 1, 3-31; He, Xiao-shu, et al. "Thiaphysovenine and Carbamate Analogues: A New Class of Potent Inhibitors of (Chlolinesterases." Medical Chemistry Research. (1992) Vol. 2, 229-237; Lahiri, D.K., *et al.* "Cholinesterase Inhibitors, β-Amyloid neurally mediated hypotension. Non-limiting examples of further active agents that can be combined with droxidopa according to the invention for use in the treatment of neurally mediated hypotension include the following:
■ corticosteroids - e.g., prednisone, cortisone, dexamethasone, methylprednisone, and fludrocortisones;
■ beta-blockers - e.g., alprenolol, carteolol, levobunolol, mepindolol, metripranolol, nadolol, oxprenolol, penbutolol, pindolol, propanolol, sotalol, timolol, acebutolol, atenolol, betaxolol, bisoprolol, esmolol, metoprolol, nebivolol, carvedilol, celiprolol, and labetalol;
■ serotonin receptor antagonist (5-HT2 and 5-HT3 antagonists) - e.g., ondansetron, tropisetron, katenserin, methysergide, cyproheptadine, and pizotifen;
■ serotonin receptor agonists (5-HTIA receptor agonists) - e.g., buspirone);
■ antiarrhythmic agents - e.g., sodium channel blockers, such as disopryamide, procainamide, quinidine, lidocaine, phenytoin, flecainide, and propafenone, potassium channel blockers, such as amiodarone, sotalol, and bretylium, and calcium channel blockers, such as verapamil and diltiazim; and
■ other various drugs that may be prescribed for treatment of neurally mediated hypotension, including but not limited to ephedrine, pseudoephedrine, theophylline, methylphenidate, and midodrine.

Although the above compounds are described in terms of classes of compounds and specific compounds, it is understood that there is substantial overlap between certain classes of compounds. Thus, specific compounds exemplifying a specific class of compounds may also properly be identified with one or more further classes of compounds. Accordingly, the above classifications should not be viewed as limiting the scope of the types of compounds useful in combination with droxidopa for treating neurally mediated hypotension.

### Il. Methods of Treatment

In one embodiment, the present invention comprises administering to a patient suffering from NMH droxidopa alone or in combination with one or more further active agents. As used herein, "treating" neurally mediated hypotension. Further, the combination of the COMT inhibitor with droxidopa may be effective for increasing the duration of the droxidopa activity (*i.e*., increasing the duration of norepinephrine activity), which may allow for a reduction in dosing frequency of the droxidopa.

The combination of droxidopa with an MAOI has a similar effect of conserving bodily norepinephrine levels. In particular embodiments, the MAOI inhibits the action of monoamine oxidase in breaking down norepinephrine, including that formed from the conversion of droxidopa. Accordingly, droxidopa plasma concentrations are positively influenced as the half-life of the droxidopa is increase. This is again particularly beneficial in allowing for reduced droxidopa dosages without limiting effective treatment of neurally mediated hypotension. Moreover, the combination of the MAOI with droxidopa is also effective for increasing droxidopa activity duration, which again may allow for a reduction in dosing frequency of the droxidopa.

In certain embodiments, the combination of droxidopa with cholinesterase inhibitors is particularly effective arising from synergistic properties. As previously noted, certain cholinesterase inhibitors (such as pyridostigmine) have been found to enhance ganglionic transmission, thereby directly affecting the function of neurotransmitters. The synergistic effect of the cholinesterase inhibitor with droxidopa can therefore be envisioned. For example, in a specific embodiment, pyridostigmine could be combined with droxidopa, the pyridostigmine enhancing ganglionic neurotransmission while the droxidopa acts to load the postganglionic neuron with norepinephrine.

The combination of droxidopa with the further active ingredients is also particularly useful in the treatment of neurally mediated hypotension. For example, combining droxidopa with one or more compounds already known to be useful in treating neurally mediated hypotension can lead to a synergistic effect. Moreover, the administration of droxidopa in combination with known treatments can lead to decreased dosing requirements for the additional agents, thus reducing any side effects associated with the additional agent. Accordingly, combining droxidopa with one or more further active agents can increase the effectiveness of treatment of neurally mediated hypotension.

Independent research has suggested a strong association between symptoms of neurally mediated hypotension and fibromyalgia. Accordingly, the invention, as described above, may particularly encompass the treatment of neurally mediated hypotension associated with fibromyalgia. Accordingly, a patient suffering from fibromyalgia and also experiencing symptoms related to neurally mediated hypotension can be treated according to the methods of the invention to achieve treatment of both the fibromyalgia-retated symptoms and the NMH-related symptoms.

Of course, the invention is not specifically limited to treatment of neurally mediated hypotension in patients also suffering from fibromyalgia. Rather, the present invention also provides methods of treatment of patients suffering from neurally mediated hypotension without comorbidities or suffering from NMH with other conditions not related to fibromyalgia. Accordingly, the present invention provides treating or preventing neurally mediated hypotension in patients susceptible to, or suffering from, NMH.

### III. Biologically Active Variants

Biologically active variants of the various compounds disclosed herein as active agents are particularly also encompassed by the invention. Such variants should retain the general biological activity of the original compounds; however, the presence of additional activities would not necessarily limit the use thereof in the present invention. Such activity may be evaluated using standard testing methods and bioassays recognizable by the skilled artisan in the field as generally being useful for identifying such activity.

According to one embodiment of the invention, suitable biologically active variants comprise analogues and derivatives of the compounds described herein. Indeed, a single compound, such as those described herein, may give rise to an entire family of analogues or derivatives having similar activity and, therefore, usefulness according to the present invention. Likewise, a single compound, such as those described herein, may represent a single family member of a greater class of compounds useful according to the present invention. Accordingly, the present invention fully encompasses not only the compounds described herein, but analogues and derivatives of such compounds, particularly those identifiable by methods commonly known in the art and recognizable to the skilled artisan.

The compounds disclosed herein as active agents may contain chiral centers, which may be either of the (R) or (S) configuration, or may comprise a mixture the phcnyl group is optionally substituted with one or more substitucnts as provided in the definition of an aryl given herein); optionally substituted arylsulfonyl; lipids (including phospholipids); phosphotidylcholine; phosphocholine; amino acid residues or derivatives; amino acid acyl residues or derivatives; peptides; cholesterols; or other pharmaceutically acceptable leaving groups which, when administered in vivo, provide the free amine and/or carboxylic acid moiety. Any of these can be used in combination with the disclosed active agents to achieve a desired effect.

### IV. Pharmaceutical Compositions

While it is possible for individual active agent compounds used in the methods of the present invention to be administered in the raw chemical form, it is preferred for the compounds to be delivered as a pharmaceutical composition. Accordingly, there arc provided by the present invention pharmaceutical compositions comprising one or more compounds described herein as active agents. As such, the compositions used in the methods of the present invention comprise the pharmaceutically active compounds, as described above, or pharmaceutically acceptable esters, amides, salts, solvates, analogs, derivatives, or prodrugs thereof. Further, the compositions can be prepared and delivered in a variety of combinations. For example, the composition can comprise a single composition containing all of the active ingredients. Alternately, the composition can comprise multiple compositions comprising separate active ingredients but intended to be administered simultaneously, in succession, or in otherwise close proximity of time.

The active agent compounds described herein can be prepared and delivered together with one or more pharmaceutically acceptable carriers therefore, and optionally, other therapeutic ingredients. Carriers should be acceptable in that they are compatible with any other ingredients of the composition and not harmful to the recipient thereof. A carrier may also reduce any undesirable side effects of the agent. Such carriers are known in the art. See, W ang et. al. (1980) J. Parent. Drug Assn. 34(6):452-462 .

Compositions may include short-term, rapid-onset, rapid-offset, controlled release, sustained release, delayed release, and pulsatile release compositions, providing the compositions achieve administration of a compound as described herein. See Remington's Pharmaceutical Sciences (18th ed.; Mack Publishing Company, Eaton, Pennsylvania, 1990) .

Pharmaceutical compositions for use in the methods of the invention are suitable for various modes of delivery, including oral, parenteral (including intravenous, intramuscular, subcutaneous, intradermal, intra-articular, intra-synovial, intrathecal, intra-arterial, intracardiac, subcutaneous, intraorbital, intracapsular, intraspinal, intrastemal, and transdermal), topical (including dermal, buccal, and sublingual), vaginal, urethral, and rectal administration. Administration can also be via nasal spray, surgical implant, internal surgical paint, infusion pump, or via catheter, stent, balloon or other delivery device. The most useful and/or beneficial mode of administration can vary, especially depending upon the condition of the recipient and the disorder being treated.

The pharmaceutical compositions may be conveniently made available in a unit dosage form, whereby such compositions may be prepared by any of the methods generally known in the pharmaceutical arts. Generally speaking, such methods of preparation comprise combining (by various methods) the active compounds of the invention with a suitable carrier or other adjuvant, which may consist of one or more ingredients. The combination of the active ingredients with the one or more adjuvants is then physically treated to present the composition in a suitable form for delivery (*e.g*., shaping into a tablet or forming an aqueous suspension).

Pharmaceutical compositions suitable for oral dosage may take various forms, such as tablets, capsules, caplets, and wafers (including rapidly dissolving or effervescing), each containing a predetermined amount of the active agent. The compositions may also be in the form of a powder or granules, a solution or suspension in an aqueous or non-aqueous liquid, and as a liquid emulsion (oil-in-water and water-in-oil). The active agents may also be delivered as a bolus, electuary, or paste. It is generally understood that methods of preparations of the above dosage forms are generally known in the art, and any such method would be suitable for the preparation of the respective dosage forms for use in delivery of the compositions according to the present invention.

In one embodiment, an active agent compound may be administered orally in combination with a pharmaceutically acceptable vehicle such as an inert diluent or an edible carrier. Oral compositions may be enclosed in hard or soft shell gelatin applied. Application can be by methods, such as airless spraying, fluidized bed coating, use of a coating pan, or the like. Materials for use as a delayed release coating can be polymeric in nature, such as cellulosic material (*e.g*., cellulose butyrate phthalate, hydroxypropyl methylcellulose phthalate, and carboxymethyl ethylcellulose), and polymers and copolymers of acrylic acid, methacrylic acid, and esters thereof.

Solid dosage forms according to the present invention may also be sustained release (*i.e*., releasing the active agents over a prolonged period of time), and may or may not also be delayed release. Sustained release compositions are known in the art and are generally prepared by dispersing a drug within a matrix of a gradually degradable or hydrolyzable material, such as an insoluble plastic, a hydrophilic polymer, or a fatty compound. Alternatively, a solid dosage form may be coated with such a material.

Compositions for parenteral administration include aqueous and non-aqueous sterile injection solutions, which may further contain additional agents, such as antioxidants, buffers, bacteriostats, and solutes, which render the compositions isotonic with the blood of the intended recipient. The compositions may include aqueous and non-aqueous sterile suspensions, which contain suspending agents and thickening agents. Such compositions for parenteral administration may be presented in unit-dose or multi-dose containers, such as, for example, sealed ampoules and vials, and may be stores in a freeze-dried (lyophilized) condition requiring only the addition of the sterile liquid carrier, for example, water (for injection), immediately prior to use. Extemporaneous injection solutions and suspensions may be prepared from sterile powders, granules, and tablets of the kind previously described.

The compositions for use in the present invention may also be administered transdermally, wherein the active agents are incorporated into a laminated structure (generally referred to as a "patch") that is adapted to remain in intimate contact with the epidermis of the recipient for a prolonged period of time. Typically, such patches arc available as single layer "drug-in-adhesive" patches or as multi-layer patches where the active agents are contained in a layer separate from the adhesive layer. Both types of patches also generally contain a hacking layer and a liner that is removed prior to attachment to the skin of the recipient. Transdermal drug delivery patches may also be comprised of a reservoir underlying the backing results obtained under analogous circumstances. The effective amount of the compositions would be expected to vary according to the weight, sex, age, and medical history of the subject. Of course, other factors could also influence the effective amount of the composition to be delivered, including, but not limited to, the specific disease involved, the degree of involvement or the severity of the disease, the response of the individual patient, the particular compound administered, the mode of administration, the bioavailability characteristics of the preparation administered, the dose regimen selected, and the use of concomitant medication. The compound is preferentially administered for a sufficient time period to alleviate the undesired symptoms and the clinical signs associated with the condition being treated. Methods to determine efficacy and dosage are known to those skilled in the art. Sec, for example, Isselbacher et al. (1996) Harrison's Principles of Internal Medicine 13 ed., 1814-1882.

In certain embodiments, a therapeutically effective amount of droxidopa comprises about 10 mg to about 3 g. Such therapeutically effective amount represents an amount of droxidopa that would be provided in a single dose when used as part of a combination according to the invention. It is understood that when the droxidopa is provided as a salt, ester, amide, or other pharmaceutically acceptable form, the amount of the pharmaceutical form of droxidopa can vary to the extent necessary to deliver a therapeutically effective amount of droxidopa. Further, as the therapeutically effective amount of droxidopa is provided as an amount for a single dose, the dosage amounts indicated herein do not necessarily represent the maximum amount of droxidopa that may be administered over the course of a 24 hour period since it is possible that multiple doses of the combination may be indicated for treatment of various conditions.

In further embodiments, the therapeutically effective amount of droxidopa can encompass varying ranges, and the appropriate range could be determined based upon the severity of the condition being treated and the one or more additional compounds with which the droxidopa is combined. In specific embodiments, a therapeutically effective amount of droxidopa comprises about 10 mg to about 2 g, about 10 mg to about 1 g, about 20 mg to about 900 mg, about 30 mg to about 850 mg, about 40 mg to about 800 mg, about 50 mg to about 750 mg, about 60 mg to about 700 mg, about 70 mg to about 650 mg, about 80 mg to about 600 mg, about mg to about 200 mg. Of course, this range is exemplary and could vary depending upon the amount of droxidopa included in the combination and the desired ratio of the compounds in the combination, as described above.

As noted above, droxidopa may also be combined with other active agents that can provide complimentary effects for the treatment of neurally mediated hypotension. When such complimentary active agents are used, they can be included in amounts typically prescribed for their respective uses.

### V. Articles of Manufacture

The present specification also includes an article of manufacture providing a composition comprising one or more active agents described herein. The article of manufacture can include a vial or other container that contains a composition suitable for use according to the present invention together with any carriers, either dried or in liquid form. In particular, the article of manufacture can comprise a kit including a container with a composition according to the invention. In such a kit, the composition can be delivered in a variety of combinations. For example, the composition can comprise a single dosage comprising all of the active ingredients. Alternately, where more than one active ingredient is provided, the composition can comprise multiple dosages, each comprising one or more active ingredients, the dosages being intended for administration in combination, in succession, or in other close proximity of time. For example, the dosages could be solid forms (*e.g*., tablets, caplets, capsules, or the like) or liquid forms (*e.g*., vials), each comprising a single active ingredient, but being provided in blister packs, bags, or the like, for administration in combination.

The article of manufacture further includes instructions in the form of a label on the container and/or in the form of an insert included in a box in which the container is packaged, for the carrying out the method of the invention. The instructions can also be printed on the box in which the vial is packaged or can be provided in a computer-readable form. The instructions contain information such as sufficient dosage and administration information so as to allow the subject or a worker in the field to administer the pharmaceutical composition. It is anticipated that a worker in the field encompasses any doctor, nurse, technician, spouse, or other caregiver that might administer the composition. The pharmaceutical composition can also be self-administered by the subject. norepinephrine. DDC inhibitors prevent the above-noted conversions and are useful in combination with precursor drugs (such as droxidopa) to focus conversion within the central nervous system and thus increase the concentration of droxidopa in the CNS.

Any compound typically recognized as inhibiting or decreasing the activity of DDC can be used according to the present invention. Non-limiting examples of DDC inhibitors useful according to the invention comprise benserazide, carbidopa, difluoromethyldopa, and α-methyldopa.

In further embodiments, an active agent used in combination with droxidopa comprises one or more compounds that at least partially inhibit the function of catechol-O-methyltransferase (such compounds being generally referred to as "COMT inhibitors"). Catechol-O-methyltransferase catalyzes the transfer of the methyl group from S-adenosyl-L-methionine to various catechol compounds (e.g., catecholamines), including dopamine, epinephrine, norepinephrine, and droxidopa. The COMT enzyme is important in the extraneuronal inactivation of catecholamines and drugs with catechol structures, and is generally one of the most important enzymes involved in the metabolism of catecholamines and their metabolites. It is present in most tissues, including the peripheral and the central nervous system.

Inhibitors of COMT slow metabolism and elimination of catechol compounds by increasing their half-life. Accordingly, COMT inhibitors can function to increase levels of naturally occurring catechol compounds, as well as alter the pharmacokinetics of administered catechol compounds (such as L-β-3,4-dihydroxyphenylalanine (L-DOPA), an immediate precursor of dopamine, generally used for symptomatic treatment of Parkinson's disease). Inhibitors of COMT can act peripherally (such as the compound entacapone), while others (such as tolcapone) are capable of crossing the blood-brain barrier and thus acting centrally and peripherally.

Any compound generally recognized as being a COMT inhibitor can be used as an additional active agent according to the invention. Non-limiting examples of COMT inhibitors useful in combination with droxidopa for treatment of neurally mediated hypotension according to the invention include the following: [(E)-2-cyano-N,N-diethyl-3-(3,4-dihydroxy-5-nitrophenyl)propenamide], also called entacapone (COMTAN^{®}); 4-dihydroxy-4'-methyl-5-nitrobenzophenone, also called tolcapone (TASMAR^{®}); and 3-(3,4-dihydroxy-5-nitrophenyl)methylene-2,4-Precursor Protein and Amyloid β-Peptides in Alzheimer's Disease." Acta Neurologica Scandinavia. (December 2000) Vol. 102 (s176), 60-67; Pei, Xue-Feng, et al. "Total Synthesis of Racemic and Optically Active Compounds Related to Physostigimine and Ring-C Heteroanalogues from 3[-2'-(Dimethylamino0ethyl]-2,3-dihydro-5-methoxy-1, 3-dimentyl-1H-indol-2-ol." Helvetica Chimica ACTA. (1994) Vol.77; Yu, Qian-sheng, et al. "Total Syntheses and Anticholinesterase Activities of (3aS)-N (8)-Norphysostigmine, (3aS)-N (8)-Norphenserine, Their Antipodal Isomers, and Other N (8)-Substituted Analogues." J. Med. Chem. (1997) Vol. 40, 2895-2901; and Yu, Q.S., et al. "Novel Phenserine-Based-Selective Inhibitors of Butyrylcholinesterase for Alzheimer's Disease." Reprinted with permission from J. Med. Chem., May 20, 1999, 42, 1855-1861.

According to yet another embodiment of the invention, an active agent used in combination with droxidopa comprises one or more compounds that at least partially inhibit the function of monoamine oxidase. Monoamine oxidase inhibitors (MAOIs) comprise a class of compounds understood to act by inhibiting the activity of monoamine oxidase, an enzyme generally found in the brain and liver of the human body, which functions to break down monoamine compounds, typically through deamination.

There are two isoforms of monoamine oxidase inhibitors, MAO-A and MAO-B. The MAO-A isoform preferentially deaminates monoamines typically occurring as neurotransmitters (e.g., serotonin, melatonin, epinephrine, norepinephrine, and dopamine). Thus, MAOIs have been historically prescribed as antidepressants and for treatment of other social disorders, such as agoraphobia and social anxiety. The MAO-B isoform preferentially deaminates phenylethylamine and trace amines. Dopamine is equally deaminated by both isoforms. MAOIs may by reversible or non-reversible and may be selective for a specific isoform. For example, the MAOI moclobemide (also known as Manerix or Aurorix) is known to be approximately three times more selective for MAO-A than MAO-B.

Any compound generally recognized as being an MAOI may be useful according to the present invention. Non-limiting examples of MAOIs useful in combination with droxidopa for preparing compositions according to the invention include the following: isocarboxazid (MARPLAN^{®}); moclobemide (Aurorix, Manerix, or Moclodura); phenelzine (NARDIL^{®}); tranylcypromine (PARNATE^{®}); selegiline (ELDEPRYL^{®}, EMSAM^{®}, or 1-deprenyl); lazabemide; nialamide; iproniazid (marsilid, iprozid, ipronid, rivivol, or propilniazida); iproclozide; toloxatone; harmala; brofaromine (Consonar); benmoxin (Neuralex); and certain tryptamines, such as 5-MeO-DMT (5-Methoxy-N,N-dimethyltryptamine) or 5-MeO-AMT (5-methoxy-α-methyltryptamine).

In specific embodiments, active agents used in combination with droxidopa comprise one or more compounds useful for treating neurally mediated hypotension or for reducing or preventing occurrence of symptoms associated with neurally mediated hypotension. As previously discussed, neurally mediated hypotension arises from a failure to maintain appropriate increases in heart rate, and the condition typically manifests itself by periods of dizziness and fainting. Other symptoms associated with neurally mediated hypotension include lightheadedness, fatigue, muscle aches, headaches, confusion, and nausea. Not surprisingly, neurally mediated hypotension often co-exists with chronic fatigue syndrome and fibromyalgia. The additional active agents of the invention can thus comprise compounds useful for treating, reducing, or preventing any of the above-noted symptoms related to neurally mediated hypotension. Moreover, the additional active agents can include compounds useful for treating, reducing, or preventing symptoms of chronic fatigue syndrome or fibromyalgia.

In certain embodiments, the present invention provides a method for treating neurally mediated hypotension comprising administering a combination of droxidopa and one or more antidepressant. Antidepressants useful according to the invention comprise selective serotonin reuptake inhibitors (SSRIs), tricyclics, serotonin norepinephrine reuptake inhibitors (5-HT-NE dual reuptake inhibitors), and norepinephrine and dopamine reuptake inhibitors (NDRIs). Non-limiting examples of specific antidepressants useful according to the invention comprise fluoxetine, paroxetine, citalopram, escitalopram, fluvoxamine, sertraline, amitriptyline, nortriptyline, desipramine, trazodone, venlafaxine, duloxetine, milnacipran, and bupropion.

The above compounds and classes of compounds are only examples of the types of active agents that can be used in combination with droxidopa for the treatment of neurally mediated hypotension and are not intended to be limiting of the invention. Rather, various further active agents can be combined with droxidopa according to the invention. Moreover, it is possible according to the invention to combine two or more additional active agents with droxidopa for the treatment of hypotension can specifically mean preventing or reducing the occurrence of an NMH episode. As previously pointed out, neurally mediated hypotension is a failure to maintain appropriate increases in blood pressure and/or heart rate that leads to dizziness and fainting. Treatment of NMH can encompass treatment of a patient that has previously been diagnosed with neurally mediated hypotension and suffers occurrences of dizziness and/or fainting. In such embodiments, treatment can comprise preventing re-occurrence of dizziness and/or fainting or can comprise reducing the occurrence of dizziness and/or fainting. Treatment of neurally mediated hypotension can also encompass treatment of patients that have not expressly been diagnosed with NMH but have been known to suffer incidences of dizziness and/or fainting that may be indicative of neurally mediated hypotension. For example, it is common for patients suffering from diseases, such as chronic fatigue syndrome and fibromyalgia, to exhibit symptoms indicative of neurally mediated hypotension. Since the patients have been diagnosed with the other condition, it is common for such patients to not be expressly diagnosed with neurally mediated hypotension. Nevertheless, such patients may also benefit from the methods of treatment described herein.

Since neurally mediated hypotension presents symptoms in addition to dizziness and fainting, treatment according to the invention can also encompass preventing or reducing occurrence of a symptom of NMH. Accordingly, the invention encompasses treating a patient suffering from neurally mediated hypotension by preventing or reducing occurrence of a symptom selected from the group consisting of dizziness, fainting, lightheadedness, fatigue, muscle aches, headaches, confusion, and nausea. In specific embodiments, treatment can evidenced by a patient being able to conduct normal daily activities (including sitting for prolonged periods, standing for prolonged periods, and intermittent sitting and standing) without exhibiting a symptom of neurally mediated hypotension. In a preferred embodiment, treatment can evidenced by a patient being able to conduct such normal daily activities without exhibiting any symptoms of neurally mediated hypotension. Preferably, the patient can conduct normal daily activities without exhibiting any symptoms for a period of at least one day, at least two days, at least three days, at least four days, at least five days, at least six days, at least one week, at least two weeks, at least three, or at least four weeks, said time frames being consecutive (e.g., at least two consecutive days, at least two consecutive weeks, etc.). In another embodiment, treatment can be evidenced by a patient being able to conduct such normal daily activities without suffering from periods of dizziness or fainting. Preferably, treatment is effective to limit any occurrence of a symptom of neurally mediated hypotension to less than one occurrence per day, less than one occurrence per week, less than one occurrence per two weeks, less than one occurrence per three weeks, or less than one occurrence per four weeks.

Neurally mediated hypotension can be diagnosed by using the so-called "tilt table test", which reveals a drop in blood pressure and may reveal a drop in heart rate. In some cases, neurally mediated hypotension is diagnosed by a drop in blood pressure alone. In other cases, such diagnosis is only made when a drop in blood pressure also accompanies a drop in heart rate. The tilt table test measures heart rate and blood pressure while lying down (during resting state), then standing at a 70 degree angle for 45 minutes. The patient is lowered while medication, such as isoproterenol, is administered through an IV to increase the heart rate to about 10% above the resting heart rate and then the patient is returned to the 70 degree angle for 15 minutes. The medication is increased to further increase the heart rate, and the patient is returned to the upright position for 10 minutes. Preferably, the test environment is quiet and non-stimulating to eliminate distractions. The patient may be required to fast after midnight prior to the test (to reduce incidence of nausea and vomiting). The patient is strapped on the table to avoid injury in case of fainting and also to decrease the human nature of compensating for the blood pooling in the legs by "fidgeting". Patient vital signs are monitored throughout the tilt table test, and the test is determined to have a positive result for NMH if there is a "significant" drop in blood pressure and a drop in heart rate. As noted above, some physicians consider a positive diagnosis with a drop in blood pressure alone.

Droxidopa is converted to norepinephrine by the action of the aromatic L-amino acid decarboxylase DDC. Although not wishing to be bound by theory, it is believed that droxidopa may be useful for treating neurally mediated hypotension because of its ability to increase norepinephrine levels via the noted conversion process. For example, the human body is known to release norepinephrine and epinephrine to cause the heart to beat and more forcefully and cause the blood vessels to tighten or constrict to compensate for times of reduced blood flow to the brain. It has been found, according to the present invention that, in a patient suffering from neurally mediated hypotension, droxidopa may be useful to increase available norepinephrine and possibly facilitate the reflex responses necessary to regulate blood pressure, heart rate, and brain blood volume, thereby preventing the onset of symptoms of NMH.

The various combinations of one or more further active ingredients with droxidopa, in certain embodiments, are particularly beneficial for treating neurally mediated hypotension. In certain embodiments, the one or more further active agents provide a conserving effect on the droxidopa. In further embodiments, the one or more further active agents provide a complimentary effect to the action of the droxidopa, preferably treating or reducing the incidence of neurally mediated hypotension.

In particular embodiments, droxidopa is combined with one or more DDC inhibitors. Such a combination is particularly beneficial for focusing the effect of the droxidopa in increasing norepinephrine levels. Many DDC inhibitors, such as benserazide and carbidopa, do not enter the central nervous system. Rather, they remain within the periphery where they prevent decarboxylation of compounds (such as levodopa or droxidopa) into the active metabolites (such as norepinephrine). Thus, when a non-CNS DDC inhibitor is administered in combination with droxidopa, the DDC inhibitor prevents decarboxylation of the droxidopa in the periphery and therefore allows more droxidopa to enter the CNS intact. Once within the CNS (and thus segregated from the DDC inhibitor), the droxidopa can be converted to norepinephrine. Accordingly, the combination of a DDC inhibitor with droxidopa can increase the effective ability of the droxidopa to provide norepinephrine within the CNS and reduce the necessary dose of droxidopa to be effective in treating neurally mediated hypotension.

As previously noted, catechol-O-methyltransferase is directly involved in the metabolism of catecholamines, including dopamine, epinephrine, norepinephrine, and droxidopa. Accordingly, by providing droxidopa in combination with a COMT inhibitor, the effect of the droxidopa to affect neurally mediated hypotension is conserved. Specifically, by inhibiting the action of COMT, the COMT inhibiting compound slows or delays the metabolism of droxidopa (as well as norepinephrine itself). This influences the overall plasma concentration of the droxidopa by increasing both the peak plasma concentration (Cₘₐₓ) and the half-life of the administered droxidopa. This is particularly beneficial in that it allows for reduced dosages of droxidopa without limiting effective treatment of neurally mediated thereof. Accordingly, the present invention also includes stereoisomers of the compounds described herein, where applicable, either individually or admixed in any proportions. Stereoisomers may include, but are not limited to, enantiomers, diastereomers, racemic mixtures, and combinations thereof. Such stereoisomers can be prepared and separated using conventional techniques, either by reacting enantiomeric starting materials, or by separating isomers of compounds of the present invention. Isomers may include geometric isomers. Examples of geometric isomers include, but are not limited to, cis isomers or trans isomers across a double bond. Other isomers are contemplated among the compounds of the present invention. The isomers may be used either in pure form or in admixture with other isomers of the compounds described herein.

Various methods are known in the art for preparing optically active forms and determining activity. Such methods include standard tests described herein other similar tests which are will known in the art. Examples of methods that can be used to obtain optical isomers of the compounds according to the present invention include the following:
i) physical separation of crystals whereby macroscopic crystals of the individual enantiomers are manually separated. This technique may particularly be used when crystals of the separate enantiomers exist (i.e., the material is a conglomerate), and the crystals are visually distinct;
ii) simultaneous crystallization whereby the individual enantiomers are separately crystallized from a solution of the racemate, possible only if the latter is a conglomerate in the solid state;
iii) enzymatic resolutions whereby partial or complete separation of a racemate by virtue of differing rates of reaction for the enantiomers with an enzyme;
iv) enzymatic asymmetric synthesis, a synthetic technique whereby at least one step of the synthesis uses an enzymatic reaction to obtain an enantiomerically pure or enriched synthetic precursor of the desired enantiomer;
v) chemical asymmetric synthesis whereby the desired enantiomer is synthesized from an achiral precursor under conditions that produce asymmetry (i.e., chirality) in the product, which may be achieved using chiral catalysts or chiral auxiliaries;
vi) diastereomer separations whereby a racemic compound is reacted with an enantiomerically pure reagent (the chiral auxiliary) that converts the individual enantiomers to diastereomers. The resulting diastereomers are then separated by chromatography or crystallization by virtue of their now more distinct structural differences and the chiral auxiliary later removed to obtain the desired enantiomer;
vii) first- and second-order asymmetric transformations whereby diastereomers from the racemate equilibrate to yield a preponderance in solution of the diastereomer from the desired enantiomer or where preferential crystallization of the diastereomer from the desired enantiomer perturbs the equilibrium such that eventually in principle all the material is converted to the crystalline diastereomer from the desired enantiomer. The desired enantiomer is then released from the diastereomers;
viii) kinetic resolutions comprising partial or complete resolution of a racemate (or of a further resolution of a partially resolved compound) by virtue of unequal reaction rates of the enantiomers with a chiral, non-racemic reagent or catalyst under kinetic conditions;
ix) enantiospecific synthesis from non-racemic precursors whereby the desired enantiomer is obtained from non-chiral starting materials and where the stereochemical integrity is not or is only minimally compromised over the course of the synthesis;
x) chiral liquid chromatography whereby the enantiomers of a racemate are separated in a liquid mobile phase by virtue of their differing interactions with a stationary phase. The stationary phase can be made of chiral material or the mobile phase can contain an additional chiral material to provoke the differing interactions;
xi) chiral gas chromatography whereby the racemate is volatilized and enantiomers are separated by virtue of their differing interactions in the gaseous mobile phase with a column containing a fixed non-racemic chiral adsorbent phase;
xii) extraction with chiral solvents whereby the enantiomers are separated by virtue of preferential dissolution of one enantiomer into a particular chiral solvent; and
xiii) transport across chiral membranes whereby a racemate is placed in contact with a thin membrane barrier. The barrier typically separates two miscible fluids, one containing the racemate, and a driving force such as concentration or pressure differential causes preferential transport across the membrane barrier. Separation occurs as a result of the non-racemic chiral nature of the membrane which allows only one enantiomer of the racemate to pass through.

The compound optionally may be provided in a composition that is enantiomerically enriched, such as a mixture of enantiomers in which one enantiomer is present in excess, in particular to the extent of 95% or more, or 98% or more, including 100%.

The compounds described herein as active agents can also be in the form of an ester, amide, salt, solvate, prodrug, or metabolite provided they maintain pharmacological activity according to the present invention. Esters, amides, salts, solvates, prodrugs, and other derivatives of the compounds of the present invention may be prepared according to methods generally known in the art, such as, for example, those methods described by J. March, Advanced Organic Chemistry: Reactions, Mechanisms and Structure, 4th Ed. (New York: Wiley-Interscience, 1992), which is incorporated herein by reference.

Examples of pharmaceutically acceptable salts of the compounds useful according to the invention include acid addition salts. Salts of non-pharmaceutically acceptable acids, however, may be useful, for example, in the preparation and purification of the compounds. Suitable acid addition salts according to the present invention include organic and inorganic acids. Preferred salts include those formed from hydrochloric, hydrobromic, sulfuric, phosphoric, citric, tartaric, lactic, pyruvic, acetic, succinic, fumaric, maleic, oxaloacetic, methanesulfonic, ethanesulfonic, p-toluenesulfonic, benzesulfonic, and isethionic acids. Other useful acid addition salts include propionic acid, glycolic acid, oxalic acid, malic acid, malonic acid, benzoic acid, cinnamic acid, mandelic acid, salicylic acid, and the like. Particular example of pharmaceutically acceptable salts include, but are not limited to, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxyenzoates, phthalates, sulfonates, xylenesulfonates, phenylacetates, phenylpropionates, phenylbutyrates, citrates, lactates, γ-hydroxybutyrates, glycolates, tartrates, methanesulfonates, propanesulfonates, naphthalene-1-sulfonates, naphthalene-2-sulfonates, and mandelates.

An acid addition salt may be reconverted to the free base by treatment with a suitable base. Preparation of basic salts of acid moieties which may be present on a compound useful according to the present invention may be prepared in a similar manner using a pharmaceutically acceptable base, such as sodium hydroxide, potassium hydroxide, ammonium hydroxide, calcium hydroxide, triethylamine, or the like.

Esters of the active agent compounds according to the present invention may be prepared through functionalization of hydroxyl and/or carboxyl groups that may be present within the molecular structure of the compound. Amides and prodrugs may also be prepared using techniques known to those skilled in the art. For example, amides may be prepared from esters, using suitable amine reactants, or they may be prepared from anhydride or an acid chloride by reaction with ammonia or a lower alkyl amine. Moreover, esters and amides of compounds of the invention can be made by reaction with a carbonylating agent (*e.g*., ethyl formate, acetic anhydride, methoxyacetyl chloride, benzoyl chloride, methyl isocyanate, ethyl chloroformate, methanesulfonyl chloride) and a suitable base (*e.g*., 4-dimethylaminopyridine, pyridine, triethylamine, potassium carbonate) in a suitable organic solvent (*e.g*., tetrahydrofuran, acetone, methanol, pyridine, N,N-dimethylformamide) at a temperature of 0 °C to 60°C. Prodrugs are typically prepared by covalent attachment of a moiety, which results in a compound that is therapeutically inactive until modified by an individual's metabolic system. Examples of pharmaceutically acceptable solvates include, but are not limited to, compounds according to the invention in combination with water, isopropanol, ethanol, methanol, DMSO, ethyl acetate, acetic acid, or ethanolamine.

In the case of solid compositions, it is understood that the compounds used in the methods of the invention may exist in different forms. For example, the compounds may exist in stable and metastable crystalline forms and isotropic and amorphous forms, all of which are intended to be within the scope of the present invention.

If a compound useful as an active agent according to the invention is a base, the desired salt may be prepared by any suitable method known to the art, including treatment of the free base with an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, pyranosidyl acids such as glucuronic acid and galacturonic acid, alpha-hydroxy acids such as citric acid and tartaric acid, amino acids such as aspartic acid and glutamic acid, aromatic acids such as benzoic acid and cinnamic acid, sulfonic acids such a p-toluenesulfonic acid or ethanesulfonic acid, or the like.

If a compound described herein as an active agent is an acid, the desired salt may be prepared by any suitable method known to the art, including treatment of the free acid with an inorganic or organic base, such as an amine (primary, secondary or tertiary), an alkali metal or alkaline earth metal hydroxide or the like. Illustrative examples of suitable salts include organic salts derived from amino acids such as glycine and arginine, ammonia, primary, secondary and tertiary amines, and cyclic amines such as piperidine, morpholine and piperazine, and inorganic salts derived from sodium, calcium, potassium, magnesium, manganese, iron, copper, zinc, aluminum and lithium.

The present invention further includes prodrugs and active metabolites of the active agent compounds described herein. Any of the compounds described herein can be administered as a prodrug to increase the activity, bioavailability, or stability of the compound or to otherwise alter the properties of the compound. Typical examples of prodrugs include compounds that have biologically labile protecting groups on a functional moiety of the active compound. Prodrugs include compounds that can be oxidized, reduced, aminated, deaminated, hydroxylated, dehydroxylated, hydrolyzed, dehydrolyzed, alkylated, dealkylated, acylated, deacylated, phosphorylated, and/or dephosphorylated to produce the active compound. In preferred embodiments, the compounds of this invention possess anti-proliferative activity against abnormally proliferating cells, or are metabolized to a compound that exhibits such activity.

A number of prodrug ligands are known. In general, alkylation, acylation, or other lipophilic modification of one or more heteroatoms of the compound, such as a free amine or carboxylic acid residue, reduces polarity and allows passage into cells. Examples of substituent groups that can replace one or more hydrogen atoms on the free amine and/or carboxylic acid moiety include, but are not limited to, the following: aryl; steroids; carbohydrates (including sugars); 1,2-diacylglycerol; alcohols; acyl (including lower acyl); alkyl (including lower alkyl); sulfonate ester (including alkyl or arylalkyl sulfonyl, such as methanesulfonyl and benzyl, wherein capsules, may be compressed into tablets or may be incorporated directly with the food of the patient's diet. The percentage of the composition and preparations may be varied; however, the amount of substance in such therapeutically useful compositions is preferably such that an effective dosage level will be obtained.

Hard capsules containing the active agent compounds may be made using a physiologically degradable composition, such as gelatin. Such hard capsules comprise the compound, and may further comprise additional ingredients including, for example, an inert solid diluent such as calcium carbonate, calcium phosphate, or kaolin. Soft gelatin capsules containing the compound may be made using a physiologically degradable composition, such as gelatin. Such soft capsules comprise the compound, which may be mixed with water or an oil medium such as peanut oil, liquid paraffin, or olive oil.

Sublingual tablets are designed to dissolve very rapidly. Examples of such compositions include ergotamine tartrate, isosorbide dinitrate, and isoproterenol HCL. The compositions of these tablets contain, in addition to the drug, various soluble excipients, such as lactose, powdered sucrose, dextrose, and mannitol. The solid dosage forms of the present invention may optionally be coated, and examples of suitable coating materials include, but are not limited to, cellulose polymers (such as cellulose acetate phthalate, hydroxypropyl cellulose, hydroxypropyl methylcellulose, hydroxypropyl methylcellulose phthalate, and hydroxypropyl methylcellulose acetate succinate), polyvinyl acetate phthalate, acrylic acid polymers and copolymers, and methacrylic resins (such as those commercially available under the trade name EUDRAGIT^{®}), zein, shellac, and polysaccharides.

Powdered and granular compositions of a pharmaceutical preparation may be prepared using known methods. Such compositions may be administered directly to a patient or used in the preparation of further dosage forms, such as to form tablets, fill capsules, or prepare an aqueous or oily suspension or solution by addition of an aqueous or oily vehicle thereto. Each of these compositions may further comprise one or more additives, such as dispersing or wetting agents, suspending agents, and preservatives. Additional excipients (*e.g*., fillers, sweeteners, flavoring, or coloring agents) may also be included in these compositions.

Liquid compositions of pharmaceutical compositions which are suitable for oral administration may be prepared, packaged, and sold either in liquid form or in the form of a dry product intended for reconstitution with water or another suitable vehicle prior to use.

A tablet containing one or more active agent compounds described herein may be manufactured by any standard process readily known to one of skill in the art, such as, for example, by compression or molding, optionally with one or more adjuvant or accessory ingredient. The tablets may optionally be coated or scored and may be formulated so as to provide slow or controlled release of the active agents.

Adjuvants or accessory ingredients for use in the compositions can include any pharmaceutical ingredient commonly deemed acceptable in the art, such as binders, fillers, lubricants, disintegrants, diluents, surfactants, stabilizers, preservatives, flavoring and coloring agents, and the like. Binders are generally used to facilitate cohesiveness of the tablet and ensure the tablet remains intact after compression. Suitable binders include, but are not limited to: starch, polysaccharides, gelatin, polyethylene glycol, propylene glycol, waxes, and natural and synthetic gums. Acceptable fillers include silicon dioxide, titanium dioxide, alumina, talc, kaolin, powdered cellulose, and microcrystalline cellulose, as well as soluble materials, such as mannitol, urea, sucrose, lactose, dextrose, sodium chloride, and sorbitol. Lubricants are useful for facilitating tablet manufacture and include vegetable oils, glycerin, magnesium stearate, calcium stearate, and stearic acid. Disintegrants, which are useful for facilitating disintegration of the tablet, generally include starches, clays, celluloses, algins, gums, and crosslinked polymers. Diluents, which are generally included to provide bulk to the tablet, may include dicalcium phosphate, calcium sulfate, lactose, cellulose, kaolin, mannitol, sodium chloride, dry starch, and powdered sugar. Surfactants suitable for use in the composition according to the present invention may be anionic, cationic, amphoteric, or nonionic surface active agents. Stabilizers may be included in the compositions to inhibit or lessen reactions leading to decomposition of the active agents, such as oxidative reactions.

Solid dosage forms may be formulated so as to provide a delayed release of the active agents, such as by application of a coating. Delayed release coatings are known in the art, and dosage forms containing such may be prepared by any known suitable method. Such methods generally include that, after preparation of the solid dosage form (*e.g*., a tablet or caplet), a delayed release coating composition is layer that is separated from the skin of the recipient by a semi-permeable membrane and adhesive layer. Transdermal drug delivery may occur through passive diffusion or may be facilitated using electrotransport or iontophoresis.

Compositions for rectal delivery include rectal suppositories, creams, ointments, and liquids. Suppositories may be presented as the active agents in combination with a carrier generally known in the art, such as polyethylene glycol. Such dosage forms may be designed to disintegrate rapidly or over an extended period of time, and the time to complete disintegration can range from a short time, such as about 10 minutes, to an extended period of time, such as about 6 hours.

Topical compositions may be in any form suitable and readily known in the art for delivery of active agents to the body surface, including dermally, buccally, and sublingually. Typical examples of topical compositions include ointments, creams, gels, pastes, and solutions. Compositions for topical administration in the mouth also include lozenges.

In certain embodiments, the compounds and compositions disclosed herein can be delivered via a medical device. Such delivery can generally be via any insertable or implantable medical device, including, but not limited to stents, catheters, balloon catheters, shunts, or coils. In one embodiment, the present invention provides medical devices, such as stents, the surface of which is coated with a compound or composition as described herein. The medical device of this invention can be used, for example, in any application for treating, preventing, or otherwise affecting the course of a disease or condition, such as those disclosed herein.

In another embodiment of the invention, pharmaceutical compositions comprising one or more active agents described herein are administered intermittently. Administration of the therapeutically effective dose may be achieved in a continuous manner, as for example with a sustained-release composition, or it may be achieved according to a desired daily dosage regimen, as for example with one, two, three, or more administrations per day. By "time period of discontinuance" is intended a discontinuing of the continuous sustained-released or daily administration of the composition. The time period of discontinuance may be longer or shorter than the period of continuous sustained-release or daily administration. During the time period of discontinuance, the level of the components of the composition in the relevant tissue is substantially below the maximum level obtained during the treatment. The preferred length of the discontinuance period depends on the concentration of the effective dose and the form of composition used. The discontinuance period can be at least 2 days, at least 4 days or at least 1 week. In other embodiments, the period of discontinuance is at least 1 month, 2 months, 3 months, 4 months or greater. When a sustained-release composition is used, the discontinuance period must be extended to account for the greater residence time of the composition in the body. Alternatively, the frequency of administration of the effective dose of the sustained-release composition can be decreased accordingly. An intermittent schedule of administration of a composition of the invention can continue until the desired therapeutic effect, and ultimately treatment of the disease or disorder, is achieved.

Administration of the composition comprises administering a pharmaceutically active agent as described herein or administering one or more pharmaceutically active agents described herein in combination with one or more further pharmaceutically active agents (*i.e*., co-administration). Accordingly, it is recognized that the pharmaceutically active agents described herein can be administered in a fixed combination (*i.e*., a single pharmaceutical composition that contains both active agents). Alternatively, the pharmaceutically active agents may be administered simultaneously (*i.e*., separate compositions administered at the same time). In another embodiment, the pharmaceutically active agents are administered sequentially (*i.e.,* administration of one or more pharmaceutically active agents followed by separate administration or one or more pharmaceutically active agents). One of skill in the art will recognized that the most preferred method of administration will allow the desired therapeutic effect.

Delivery of a therapeutically effective amount of a composition according to the invention may be obtained via administration of a therapeutically effective dose of the composition. Accordingly, in one embodiment, a therapeutically effective amount is an amount effective to treat neurally mediated hypotension. In another embodiment, a therapeutically effective amount is an amount effective to treat a symptom of neurally mediated hypotension. Moreover, a therapeutically effective amount can be an amount effective to prevent the onset of neurally mediated hypotension in individuals susceptible to NMH. For example, as noted previously, patients suffering from chronic fatigue syndrome and fibromyalgia often also exhibit or develop symptoms of neurally mediated hypotension. Accordingly, it is possible according to the invention to treat a patient suffering from chronic fatigue syndrome or fibromyalgia (and is thus susceptible to neurally mediated hypotension) by administering a composition according to the invention and thus prevent the onset of symptoms of neurally mediated hypotension.

The active agents included in the pharmaceutical composition are present in an amount sufficient to deliver to a patient a therapeutic amount of an active ingredient *in vivo* in the absence of serious toxic effects. The concentration of active agent in the drug composition will depend on absorption, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the dosage ranges set forth herein are exemplary only and are not intended to limit the scope or practice of the claimed composition. The active ingredient may be administered at once, or may be divided into a number of smaller doses to be administered at varying intervals of time

A therapeutically effective amount according to the invention can be determined based on the bodyweight of the recipient. Alternatively, a therapeutically effective amount can be described in terms of a fixed dose. In still further embodiments, a therapeutically effective amount of one or more active agents disclosed herein can be described in terms of the peak plasma concentration achieved by administration of the active agents. Of course, it is understood that the therapeutic amount could be divided into a number of fractional dosages administered throughout the day. The effective dosage range of pharmaceutically acceptable salts and prodrugs can be calculated based on the weight of the parent nucleoside to be delivered. If a salt or prodrug exhibits activity in itself, the effective dosage can be estimated as above using the weight of the salt or prodrug, or by other means known to those skilled in the art.

It is contemplated that compositions of the invention comprising one or more active agents described herein will be administered in therapeutically effective amounts to a mammal, preferably a human. An effective dose of a compound or composition for treatment of any of the conditions or diseases described herein can be readily determined by the use of conventional techniques and by observing 90 mg to about 550 mg, about 100 mg to about 500 mg, about 100 mg to about 400 mg, or about 100 mg to about 300 mg.

In yet other embodiments, a therapeutically effective amount of droxidopa can be even greater, such as when provided as a sustained-, extended-, or continuous-release formulation. As understood in the art, such formulations provide an increased drug amount in a single dosage form that slowly releases the drug over time. A therapeutically effective amount of droxidopa for use in such a formulation can be calculated in light of the effective amounts described above and the determined frequency of dosing that would otherwise be necessary to treat a given condition.

A therapeutically effective amount of the one or more additional compounds that are combined with droxidopa according to the invention can be determined in relation to the amount of droxidopa included in the dosage form and the desired ratio of droxidopa to the additional compound(s). Advantageously, the present invention allows for great flexibility in formulating combinations. For example, the conserving effects provided by the one or more additional compounds can allow for using droxidopa in a lesser amount and still achieve the same, or better, therapeutic effects achieved using droxidopa alone. Likewise, it is possible to increase the therapeutic effects of droxidopa by using an amount of the one or more additional compounds that is less than the typically recommended dosage for the one or more additional compounds.

In one embodiment, the ratio of droxidopa to the one or more additional compounds is in the range of about 500:1 to about 1:10. In further embodiments, the ratio of droxidopa to the additional compound(s) is in the range of about 250:1 to about 1:5, about 100:1 1 to about 1:2, about 80:1 to about 1:1, about 50:1 to about 2:1, or about 20:1 to about 3:1.

The one or more additional compounds combined with droxidopa according to the invention can be included in amount typically recommended for use of the compounds alone for other indications. However, as noted above, it is possible according to the invention to use the additional compound(s) in amounts that are less than typically recommended, particularly in relation to DDC inhibitors, COMT inhibitors, cholinesterase inhibitors, and MAO inhibitors. In certain embodiments, a therapeutically effective amount of a DDC inhibitor, COMT inhibitor, cholinesterase inhibitor, or MAO inhibitor to be combined with droxidopa is in the range of about 1 EXPERIMENTAL

The present invention will now be described with specific reference to various examples. The following examples are not intended to be limiting of the invention and are rather provided as exemplary embodiments.

### EXAMPLE 1

### Pharmacokinetic Properties of Droxidopa Combinations

The pharmacokinetic properties of droxidopa combinations useful in the methods of the invention were evaluated in male Sprague Dawley rats. Four test groups with four rats in each group were established. Group 1 was administered droxidopa alone as a baseline group. Group 2 was administered droxidopa in combination with the COMT inhibitor entacapone. Group 3 was administered droxidopa in combination with the cholinesterase inhibitor pyridostigmine. Group 4 was administered droxidopa in combination with the MAOI nialamide. For each group, the droxidopa or droxidopa combination was formulated with a vehicle formed of a water solution containing 1% carboxymethylcellulose with 0.2% TWEEN^{®} 80 emulsifier.

The weights and of the droxidopa, entacapone, pyridostigmine, nialamide, and the vehicle provided in the various formulations are shown in Table 1. The calculated concentrations for each component are separately provided in Table 2. The amounts of entacapone, pyridostigmine, and nialamide used in formulations 2-7 were provided as "low" doses and "high" doses based upon disclosure in the literature of generally accepted dosage ranges for their respective known indications.

**Table 1**

| Formulation | Formulation Components - weight (g) | | | | |
|---|---|---|---|---|---|
| | Vehicle | Droxidopa | Entacapone | Pyridostigmine | Nialamide |
| 1 | 13.87 g | 0.280 g | | | |
| 2 | 13.65 g | 0.280 g | 0.0084 g | | |
| 3 | 13.60 g | 0.280 g | 0.0842 g | | |
| 4 | 13.53 g | 0.280 g | | 0.0028 g | |
| 5 | 13.60 g | 0.280 g | | 0.0563 g | |
| 6 | 13.61 g | 0.280 g | | | 0.0028 g |
| 7 | 13.70 g | 0.280 g | | | 0.0842 g |

**Table 2**

| Formulation | Formulation Components - concentration (mg/g) | | | |
|---|---|---|---|---|
| | Droxidopa | Entacapone | Pyridostigmine | Nialamide |
| 1 | 19.81 mg/g | | | |
| 2 | 20.11 mg/g | 0.603 mg/g | | |
| 3 | 20.08 mg/g | 6.031 mg/g | | |
| 4 | 20.30 mg/g | | 0.203 mg/g | |
| 5 | 20.20 mg/g | | 4.042 mg/g | |
| 6 | 20.15 mg/g | | | 0.202 mg/g |
| 7 | 20.04 mg/g | | | 5.985 mg/g |

Rats in each group were given a single gavage dose of droxidopa alone or the droxidopa combination, the time of dosing being recorded as time = 0. Dosing was based upon the weight of the subject and was adjusted to provide all test subjects a droxidopa dose of approximately 100 mg per kg of body weight. Blood samples (approximately 100 µL) were collected at approximately 5, 15, and 30 minutes, and at approximately 1, 2, 4, 8, and 24 hours after dosing. Dosing and blood collection were via an indwelling jugular vein cannula. Blood samples were drawn into a heparinized 1 mL syringe (charged with 5 µL of heparin solution [1000 U/mL]) and then transferred to a microcentrifuge.

Acetonitrile (100 µL) containing 0.2% formic acid was added to 25 µL of each plasma sample in a microcentrifuge tube. Internal standard (5 µL of 4 µg/mL 3,4-dihydroxybenzylamine (DHBA) in acetonitrile) was added, and the samples were vortexed and centrifuged to precipitate protein. The supernatant was transferred to an autosample vial with insert and injected on an Applied Biosystems API 4000 Liquid Chromatography-Mass Spectrometer (LC-MS) apparatus interfaced with an Agilent 100 High Pressure Liquid Chromatography (HPLC) apparatus. Data was collected and processed using Analyst software. The autosampler was cooled to 4 °C, and the sample injection volume 5 µL. Chromatography was conducted on a Waters Atlantis dC18 column (25 cm x 4.6 mm, 5 µm) with guard column. The solvent was water containing 0.2% formic acid, and the flow rate was set at 0.8 mL/min.

Plasma droxidopa concentration in the rat test subjects following administration of droxidopa alone or droxidopa combinations according to the invention is provided in Table 4. As a standard, plasma droxidopa concentration in rats administered the drug vehicle with no droxidopa or droxidopa combinations present was also evaluated, and no droxidopa was detected over a 24 hour period in plasma from the rats administered the vehicle alone. Likewise, no droxidopa was detected in any subjects prior to dosing of the droxidopa or droxidopa combinations. As seen in Table 3, plasma droxidopa concentration reached a maximum concentration for all formulations in a time of approximately 1-2 hours following dosing.

**Table 3**

| | Mean Plasma Droxidopa Concentration (µg/mL) at Time Post-Dosage | | | | | | | |
|---|---|---|---|---|---|---|---|---|
| Formulation | 0.083 hr | 0.25 hr | 0.5 hr | 1 hr | 2 hr | 4 hr | 8 hr | 24 hr |
| 1 | 0.401 | 3.996 | 7.869 | 11.336 | 10.548 | 3.391 | 0.610 | 0.001 |
| 2 | 0.328 | 3.245 | 8.641 | 12.050 | 8.772 | 4.795 | 3.054 | 0.005 |
| 3 | 0.189 | 2.799 | 6.775 | 8.440 | 9.270 | 3.425 | 1.853 | 0.010 |
| 4 | 0.459 | 3.570 | 7.941 | 10.054 | 8.650 | 2.976 | 1.795 | 0.005 |
| 5 | 0.456 | 4.033 | 7.341 | 8.380 | 5.989 | 2.429 | 0.431 | 0.002 |
| 6 | 0.493 | 2.867 | 6.807 | 8.579 | 6.065 | 1.829 | 0.297 | 0.000 |
| 7 | 0.311 | 3.017 | 6.506 | 7.886 | 6.381 | 2.380 | 1.535 | 0.113 |

Administration of droxidopa combinations was seen to affect plasma norepinephrine concentration in comparison to administration of droxidopa alone. Mean plasma norepinephrine concentration at 2 hours post-dosing with the various formulations tested is provided in Table 4. Formulation 0 indicates administration of the vehicle alone without droxidopa or a droxidopa combination of the invention and provides a baseline comparative of plasma norepinephrine levels in an untreated subject.

**Table 4**

| Formulation | Plasma Norepinephrine Concentration (pg/µL) |
|---|---|
| 0 | 0.711 |
| 1 | 3.320 |
| 2 | 3.358 |
| 3 | 6.359 |
| 4 | 4.000 |
| 5 | 2.290 |
| 6 | 2.182 |
| 7 | 2.674 |

As seen in Table 4, administration of droxidopa alone caused an approximate 5-fold increase in plasma norepinephrine concentration. Treatment with droxidopa in combination with the COMT inhibiting compound caused an even greater increase in plasma norepinephrine concentration. Treatment with droxidopa in combination with a relatively low dose of the cholinesterase inhibiting compound similarly caused an increase in plasma norepinephrine concentration in relation to treatment with droxidopa alone; however, the plasma norepinephrine concentration was reduced in relation to treatment with droxidopa alone when a combination of droxidopa with a relatively high dose of the cholinesterase inhibiting compound was used. Plasma norepinephrine concentration after treatment with both combinations of droxidopa with the MAOI compound was reduced relative to treatment with droxidopa alone.

Mean values for various pharmacokinetic properties of the inventive combinations used in the above study are provided in Table 5. Specifically, Table 5 provides the terminal elimination half-life (T_{1/2}) of the administered formulations, the maximum observed concentration (Cₘₐₓ) of the active agents in each formulation, the time to reach the maximum observed concentration (Tₘₐₓ), the area under the plasma concentration-time curve from time zero to the last measured time point (AUCₐₗₗ), and the observed volume of distribution at steady state (Vz_F_obs). Note that for extravascular models, the fraction of dose absorbed cannot be estimated. Therefore, Vz_F_obs for such models is actually Volume/F where F is the fraction of dose absorbed.

**Table 5**

| Formulation | T_{1/2} (hr) | Cₘₐₓ (µg/mL) | Tₘₐₓ (hr) | AUCₐₗₗ (hr µg/mL) | Vz_F_obs (mL/kg) |
|---|---|---|---|---|---|
| 1 | 1.4 | 11.4 | 1.25 | 44.4 | 5270.4 |
| 2 | 1.86 | 12.4 | 1.125 | 71.1 | 5016.4 |
| 3 | 2.64 | 10.2 | 1.38 | 52.3 | 8854.1 |
| 4 | 1.93 | 10.1 | 1.25 | 51.2 | 5900.8 |
| 5 | 1.79 | 8.4 | 1 | 30.5 | 8763.6 |
| 6 | 1.41 | 8.6 | 1 | 27.2 | 8030.0 |
| 7 | 3.77 | 8.1 | 0.875 | 42.0 | 16404.1 |

As seen above, when droxidopa is combined with certain additional active agents, the combination can increase the half-life of droxidopa, and such increase can be seen in a variety of pathways, such as through an effect on drug metabolism, volume of distribution of the drug, or a combination of the two. For example, the increase in half-life arising from the combination with entacapone indicates peripheral activity to block the metabolism of droxidopa to 3-OM-droxidopa (the major metabolite of droxidopa), thus increasing residence time of droxidopa in the body. Similarly, an increase in the volume of distribution indicates a decrease in the amount of drug available to organs of elimination, which can further affect half-life. The increase in half-life associated with the relatively high dose of nialamide is surprising since MAOIs are not typically considered to be a major metabolic pathway for droxidopa, and is likely the result of the unexpected increase in the apparent volume of distribution. Similarly, the combination with pyridostigmine also surprisingly led to an increase in droxidopa half-life even though cholinesterase compounds would generally not be expected to affect droxidopa metabolism. Droxidopa half-life when administered alone or in combination with entacapone, pyridostigmine, or nialamide is graphically illustrated in FIG. 1.

## Claims

1. A therapeutically effective amount of droxidopa for use for treating neurally mediated hypotension in a patient.

2. The therapeutically effective amount of droxidopa for use according to claim 1, wherein the patient suffering from neurally mediated hypotension exhibits a symptom selected from the group consisting of dizziness, fainting, lightheadedness, fatigue, muscle aches, headaches, confusion, and nausea, and wherein the therapeutically effective amount is sufficient to reduce or eliminate the symptom.

3. The therapeutically effective amount of droxidopa for use according to claim 1, wherein the patient suffering from neurally mediated hypotension has previously exhibited a symptom selected from the group consisting of dizziness, fainting, lightheadedness, fatigue, muscle aches, headaches, confusion, and nausea, and wherein the therapeutically effective amount is sufficient to prevent re-occurrence of the symptom.

4. The therapeutically effective amount of droxidopa for use according to claim 1, wherein the therapeutically effective amount is sufficient to reduce or eliminate occurrence of an episode of dizziness or fainting associated with neurally mediated hypotension.

5. The therapeutically effective amount of droxidopa for use according to claim 4, wherein dizziness or fainting associated with neurally mediated hypotension is reduced such that an episode of dizziness or fainting is reduced to less than one occurrence per day.

6. The therapeutically effective amount of droxidopa for use according to claim 4, wherein dizziness or fainting associated with neurally mediated hypotension is reduced such that an episode of dizziness or fainting is reduced to less than one occurrence per week.

7. The therapeutically effective amount of droxidopa for use according to claim 1, wherein the therapeutically effective amount is sufficient such that the patient can conduct normal daily activities without exhibiting any symptoms of neurally mediated hypotension for a period of at least one week.

8. The therapeutically effective amount of droxidopa for use according to claim 1, further comprising administering one or more additional active agents selected from the group consisting of DOPA decarboxylase inhibiting compounds, catechol-O-methyltransferase inhibitors, cholinesterase inhibitors, antidepressants, corticosteroids, beta-blockers, serotonin receptor antagonists, serotonin receptor agonists, antiarrhythmic agents, ephedrine, pseudoephedrine, theophylline, methylphenidate, midodrine, and combinations thereof.

9. The therapeutically effective amount of droxidopa for use according to claim 8, wherein the one or more additional active agents comprise one or more DOPA decarboxylase inhibiting compounds selected from the group consisting of benserazide, carbidopa, difluoromethyldopa, α-methyldopa, and combinations thereof.

10. The therapeutically effective amount of droxidopa for use according to claim 8, wherein the one or more additional active agents comprise one or more catechol-O-methyltransferase inhibitors selected from the group consisting of entacapone, tolcapone, nitecapone, and combinations thereof.

11. The therapeutically effective amount of droxidopa for use according to claim 8, wherein the one or more additional active agents comprise one or more cholinesterase inhibitors selected from the group consisting of pyridostigmine, donepezil, rivastigmine, galantamine, tacrine, neostigmine, metrifonate, physostigmine, ambenonium, edrophonium, demarcarium, thiaphysovenine, phenserine, cymserine, and combinations thereof.

12. The therapeutically effective amount of droxidopa for use according to claim 8, wherein the one or more additional active agents comprise one or more monoamine oxidase inhibitors selected from the group consisting of isocarboxazid, moclobemide, phenelzine, tranylcypromine, selegiline, lazabemide, nialamide, iproniazid, iproclozide, toloxatone, harmala, brofaromine, benmoxin, 5-Methoxy-N,N-dimethyltryptamine, 5-methoxy-α-methyltryptamine, and combinations thereof.

13. The therapeutically effective amount of droxidopa for use according to claim 8, wherein the one or more additional active agents comprise one or more antidepressants selected from the group consisting of selective serotonin reuptake inhibitors, tricyclics, serotonin norepinephrine reuptake inhibitors, norepinephrine reuptake inhibitors, norepinephrine and dopamine reuptake inhibitors, and combinations thereof.

14. The therapeutically effective amount of droxidopa for use according to claim 13, wherein the antidepressants are selected from the group consisting of fluoxetine, paroxetine, citalopram, escitalopram, fluvoxamine, sertraline, amitriptyline, nortriptyline, desipramine, trazodone, venlafaxine, duloxetine, milnacipran, nefopam, bupropion, and combinations thereof.

15. The therapeutically effective amount of droxidopa for use according to claim 8, wherein the one or more additional active agents are formulated in the same pharmaceutical composition with droxidopa.

16. The therapeutically effective amount of droxidopa for use according to claim 8, wherein the one or more additional active agents are administered in a pharmaceutical composition separate from droxidopa.

## Patentansprüche

1. Therapeutisch wirksame Menge von Droxidopa zur Verwendung zur Behandlung von neural vermittelter Hypotonie bei einem Patienten.

2. Therapeutisch wirksame Menge von Droxidopa zur Verwendung gemäß Anspruch 1, wobei der Patient, der an neural vermittelter Hypotonie leidet, ein Symptom aufweist, das aus der Gruppe, bestehend aus Schwindel, Ohnmacht, Benommenheit, Müdigkeit, Muskelschmerzen, Kopfweh, Verwirrung und Nausea, ausgewählt ist, und wobei die therapeutisch wirksame Menge ausreichend ist, um das Symptom zu verringern oder zu eliminieren.

3. Therapeutisch wirksame Menge von Droxidopa zur Verwendung gemäß Anspruch 1, wobei der Patient, der an neural vermittelter Hypotonie leidet, vorher ein Symptom aufwies, das ausgewählt ist aus der Gruppe, bestehend aus Schwindel, Ohnmacht, Benommenheit, Müdigkeit, Muskelschmerzen, Kopfweh, Verwirrung und Nausea, und wobei die therapeutisch wirksame Menge ausreichend ist, um ein Wiederauftreten des Symptoms zu verhindern.

4. Therapeutisch wirksame Menge von Droxidopa zur Verwendung gemäß Anspruch 1, wobei die therapeutisch wirksame Menge ausreichend ist, um das Auftreten einer Episode von Schwinden oder Ohnmacht, assoziiert mit neural vermittelter Hypotonie, zu verringern oder zu eliminieren,

5. Therapeutisch wirksame Menge von Droxidopa zur Verwendung gemäß Anspruch 4, wobei Schwindel oder Ohnmacht, assoziiert mit neural vermittelter Hypotonie, verringert wird, sodass eine Episode von Schwindel oder Ohnmacht auf weniger als ein Vorkommen pro Tag verringert wird.

6. Therapeutisch wirksame Menge von Droxidopa zur Verwendung gemäß Anspruch 4, wobei Schwindel oder Ohnmacht, assoziiert mit neural vermittelter Hypotonie, so verringert wird, dass eine Episode von Schwindel oder Ohnmacht auf weniger als ein Vorkommen pro Woche verringert wird.

7. Therapeutisch wirksame Menge von Droxidopa zur Verwendung gemäß Anspruch 1, wobei die therapeutisch wirksame Menge ausreichend ist, sodass der Patient normale Tagesaktivitäten ohne Auftreten irgendwelcher Symptome von neural vermittelter Hypotonie für einen Zeitraum von wenigstens einer Woche durchführen kann.

8. Therapeutisch wirksame Menge von Droxidopa zur Verwendung gemäß Anspruch 1, die außerdem ein Verabreichen eines zusätzlichen aktiven Mittels oder mehrer zusätzlicher aktiver Mittel, ausgewählt aus der Gruppe, bestehend aus DOPA-Decarboxylase-inhibierenden Verwindungen, Catechol-O-Methyltransferase-Inhibitoren, Cholinesterase-Inhibitoren, Antidepressiva, Corticosteroiden, Betablockern, Serotoninrezeptor-Antagonisten, Serotoninrezeptor-Agonisten, Antiarrhytmika, Ephedrin, Pseudoephedrin, Theophyllin, Methylphenidat, Midodrin und Kombinationen davon, umfasst.

9. Therapeutisch wirksame Menge von Droxidopa zur Verwendung gemäß Anspruch 8, wobei das eine zusätzliche aktive Mittel oder die mehreren zusätzlichen aktiven Mittel eine oder mehrere DOPA-Decarboxylase-inhibierende Verbindung(en), ausgewählt aus der Gruppe, bestehend aus Benserazid, Carbidopa, Difluormethyldopa, α-Methyldopa und Kombinationen davon, umfasst/umfassen.

10. Therapeutisch wirksame Menge von Droxidopa zur Verwendung gemäß Anspruch 8, wobei das eine zusätzliche aktive Mittel oder die mehreren zusätzlichen aktiven Mittel einen oder mehrere Catechol-O-Methyltransferase-Inhibitor(en), ausgewählt aus der Gruppe, bestehend aus Entacapon, Tolcapon, Nitecapon und Kombinationen davon, umfasst/umfassen.

11. Therapeutisch wirksame Menge von Droxidopa zur Verwendung gemäß Anspruch 8, wobei das eine zusätzliche aktive Mittel oder die mehreren zusätzlichen aktiven Mittel einen oder mehrere Cholinesterase-Inhibitor(en) ausgewählt aus der Gruppe, bestehend aus Pyridostigmin, Donepezil, Rivastigmin, Galantamin, Tacrin, Neostigmin, Metrifonat, Physcstigmin, Ambenonium, Edrophonium, Demarcarium, Thiaphysovenin, Phenserin, Cymserin und Kombinationen davon, umfasst/umfassen.

12. Therapeutisch wirksame Menge von Droxidopa zur Verwendung gemäß Anspruch 8, wobei das eine zusätzliche aktive Mittel oder die mehreren zusätzlichen aktiven Mittel einen oder mehrere Monoaminoxidase-Inhibitor(en), ausgewählt aus der Gruppe bestehend aus Isocarboxazid, Moclobemid, Phenelzin, Tranylcypromin, Selegilin, Lazabemid, Nialamid, Iproniazid, Iproclozid, Toloxaton, Harmala, Brofaromin, Benmoxin, 5-Methoxy-N,N-dimethyltryptamin, 5-Methoxy-α-methyltryptamin und Kombinationen davon, umfasst/umfassen.

13. Therapeutisch wirksame Menge von Droxidopa zur Verwendung gemäß Anspruch 8, wobei das eine zusätzliche aktive Mittel oder die mehreren zusätzlichen aktiven Mittel ein Antidepressivum oder mehrere Antidepressiva, ausgewählt aus der Gruppe, bestehend aus selektiven Serotonin-Wiederaufnahme-Inhibitoren, trizyklischen Mitteln, Serotonin-Norepinephrin-Wiederaufnahme-Inhibitoren, Norepinephrin-Wiederaufnahme-Inhibitoren, Norepinephrin- und Dopamin-Wiederaufnahme-Inhibitoren und Kombinationen davon, umfasst/umfassen.

14. Therapeutisch wirksame Menge von Droxidopa zur Verwendung gemäß Anspruch 13, wobei die Antidepressiva aus der Gruppe, bestehend aus Fluoxetin, Paroxetin, Citalopram, Escitalopram, Fluvoxamin, Sertralin, Amitryptylin, Nortryptylin, Desipramin, Trazodon, Vonlafaxin, Duloxctin, Milnacipran, Nefopam, Bupropion und Kombinationen davon, ausgewählt sind.

15. Therapeutisch wirksame Menge von Droxidopa zur Verwendung gemäß Anspruch 8, wobei das eine zusätzliche aktive Mittel oder die mehreren zusätzlichen aktiven Mittel in der gleichen pharmazeutischen Zusammensetzung mit Droxidopa formuliert sind.

16. Therapeutisch wirksame Menge von Droxidopa zur Verwendung gemäß Anspruch 8, wobei das eine zusätzliche aktive Mittel oder die mehreren zusätzlichen aktiven Mittel in einer von Droxidopa getrennten pharmazeutischen Zusammensetzung verabreicht wird/werden.

## Revendications

1. Quantité thérapeutiquement efficace de droxidopa destinée à être utilisée dans le traitement de l'hypotension à médiation neurorale chez un patient.

2. Quantité thérapeutiquement efficace de droxidopa destinée à être utilisée selon la revendication 1, dans laquelle le patient souffrant d'une hypotension à médiation neuronale présente un symptôme choisi dans le groupe consistant en les vertiges, les évanouissements, les étourdissements, la fatigue, les douleurs musculaires, les maux de tête, la confusion, et la nausée, et dans laquelle la quantité thérapeutiquement efficace est suffisante pour réduire ou éliminer les symptômes.

3. Quantité thérapeutiquement efficace de droxidopa destinée à être utilisée selon la revendication 1, dans laquelle le patient souffrant d'une hypotension à médiation neuronale a précédemment présenté un symptôme choisi dans le groupe consistant en les vertiges, les évanouissements, les étourdissements, la fatigue, les douleurs musculaires, les maux de tête, la confusion, et la nausée, et dans laquelle la quantité thérapeutiquement efficace est suffisante pour prévenir la réapparition des symptômes.

4. Quantité thérapeutiquement efficace de droxidopa destinée à être utilisée selon la revendication 1, dans laquelle la quantité thérapeutiquement efficace est suffisante pour réduire ou éliminer l'apparition d'un épisode de vertige ou d'évanouissement associé à l'hypotension à médiation neuronale.

5. Quantité thérapeutiquement efficace de droxidopa destinée à être utilisée selon la revendication 4, dans laquelle les vertiges ou les évanouissements associés à l'hypotension à médiation neuronale sont réduits de sorte qu'un épisode de vertige ou d'évanouissement est réduit à moins d'une apparition par jour.

6. Quantité thérapeutiquement efficace de droxidopa destinée à être utilisée selon la revendication 4, dans laquelle les vertiges ou les évanouissements associés à l'hypotension à médiation neuronale sont réduits de sorte qu'un épisode de vertige ou d'évanouissement est réduit à moins d'une apparition par semaine.

7. Quantité thérapeutiquement efficace de droxidopa destinée à être utilisée selon la revendication 1, dans laquelle la quantité thérapeutiquement efficace est suffisante de sorte que le patient puisse poursuivre ses activités quotidiennes normales sans présenter aucun symptôme d'hypotension à médiation neuronale pendant une période d'au moins une semaine.

8. Quantité thérapeutiquement efficace de droxidopa destinée à être utilisée selon la revendication 1, comprenant en outre l'administration d'un ou de plusieurs agents actifs supplémentaires choisis dans le groupe consistant en les composés inhibant la DOPA décarboxylase, les inhibiteurs de la catéchol-O-méthyltransférase, les inhibiteurs de la cholinestérase, les antidépresseurs, les corticostéroïdes, les composés bêtabloquants, les antagonistes des récepteurs de la sérotonine, les agonistes des récepteurs de la sérotonine, les agents anti-arythmisants, l'éphédrine, la pseudoéphédrine, la théophylline, le méthylphénidate, la midodrine, et des combinaisons de ceux-ci.

9. Quantité thérapeutiquement efficace de droxidopa destinée à être utilisée selon la revendication 8, dans laquelle l'un ou plusieurs agents actifs supplémentaires comprennent un ou plusieurs composés inhibant la DOPA décarboxylase choisis dans le groupe consistant en le bensérazide, la carbidopa, la difluorométhyldopa, l'α-méthyldopa, et des combinaisons de ceux-ci.

10. Quantité thérapeutiquement efficace de droxidopa destinée à être utilisée selon la revendication 8, dans laquelle l'un ou plusieurs agents actifs supplémentaires comprennent un ou plusieurs inhibiteurs de la catéchol-O-méthyltransférase choisis dans le groupe consistant en l'entacapone, la tolcapone, la nitécapone, et des combinaisons de ceux-ci.

11. Quantité thérapeutiquement efficace de droxidopa destinée à être utilisée selon la revendication 8, dans laquelle le ou les agents actifs supplémentaires comprennent un ou plusieurs inhibiteurs de la cholinestérase choisis dans le groupe consistant en la pyridostigmine, le donépézil, la rivastigmine, la galantamine, la tacrine, la néostigmine, le métrifonate, la physostigmine, l'ambénonium, l'édrophonium, le démarcarium, la thiaphysovénine, la phensérine, la cymsérine, et des combinaisons de ceux-ci.

12. Quantité thérapeutiquement efficace de droxidopa destinée à être utilisée selon la revendication 8, dans laquelle l'un ou plusieurs agents actifs supplémentaires comprennent un ou plusieurs inhibiteurs de la monoamine oxydase choisis dans le groupe consistant en l'isocarboxazide, le moclobémide, la phénelzine, la tranylcypromine, la sélégiline, le lazabémide, le nialamide, l'iproniazide, l'iproclozide, la toloxatone, l'harmala, la brofaromine, la benmoxine, la 5-méthoxy-N,N-diméthyltryptamine, la 5-méthoxy-α-méthyltryptamine, et des combinaisons de ceux-ci.

13. Quantité thérapeutiquement efficace de droxidopa destinée à être utilisée selon la revendication 8, dans laquelle l'un ou plusieurs agents actifs supplémentaires comprennent un ou plusieurs antidépresseurs choisis dans le groupe consistant en les inhibiteurs sélectifs de la recapture de la sérotonine, des agents tricycliques, des inhibiteurs de la recapture de la sérotonine et de la norépinéphrine, les inhibiteurs de la recapture de la norépinéphrine, les inhibiteurs de la recapture de la norépinéphrine et de la dopamine, et des combinaisons de ceux-ci.

14. Quantité thérapeutiquement efficace de droxidopa destinée à être utilisée selon la revendication 13, dans laquelle les antidépresseurs sont choisis dans le groupe consistant en la fluoxétine, la paroxétine, le citalopram, l'escitalopram, la fluvoxamine, la sertraline, l'amitriptyline, la nortriptyline, la désipramine, la trazodone, la venlafaxine, la duloxétine, le milnacipran, le néfopam, le bupropion, et des combinaisons de ceux-ci.

15. Quantité thérapeutiquement efficace de droxidopa destinée à être utilisée selon la revendication 8, dans laquelle l'un ou plusieurs agents actifs supplémentaires sont formulés dans la même composition pharmaceutique comprenant la droxidopa.

16. Quantité thérapeutiquement efficace de droxidopa destinée à être utilisée selon la revendication 8, dans laquelle l'un ou plusieurs agents actifs supplémentaires sont administrés dans une composition pharmaceutique à part de celle comprenant la droxidopa.
